# EUROPÄISCHE PATENTANMELDUNG

(11) **EP 2 905 334 A1**
(43) Veröffentlichungstag der Anmeldung: **12.08.2015**
(21) Anmeldenummer: 15151885.9
(22) Anmeldetag: 21.01.2015
(51) Int. Cl.: C12N 9/96, C12N 9/99

(54) **Enzymsystem, Kit und Verfahren zur Durchführung von enzymatischen Reaktionen**

(30) Priorität: 11.02.2014 DE 102014202379
(71) Anmelder: ROBERT BOSCH GMBH, 70442 Stuttgart (DE)
(72) Erfinder: Daub, Martina, 71287 Weissach (DE); Faltin, Bernd, 70839 Gerlingen (DE); Dorrer, Christian, 70193 Stuttgart (DE)

(57) **Zusammenfassung**

Bei einem Enzymsystem sind wenigstens ein erstes Enzym (11) und wenigstens ein zweites Enzym (12) enthalten, wobei die enzymatische Aktivität des wenigstens zweiten Enzyms (12) eine Inaktivierung des wenigstens ersten Enzyms (11) bewirkt. Erfindungsgemäß wird die Aktivität des wenigstens zweiten Enzyms (12) durch eine Temperierung gesteuert. In einer ersten Inkubationsphase kann das erste Enzym (11) seine enzymatische Aktivität entfalten. In einer zweiten Inkubationsphase dominiert die enzymatische Aktivität des zweiten Enzyms (12), wodurch das erste Enzym inaktiviert wird und die Reaktion damit beendet werden kann.

## Beschreibung

Die vorliegende Erfindung betrifft ein Enzymsystem mit wenigstens einem ersten Enzym und wenigstens einem zweiten Enzym, wobei das wenigstens zweite Enzym zur Inaktivierung des wenigstens ersten Enzyms vorgesehen ist, sowie einen Kit zur Durchführung von enzymatischen Reaktionen und ein entsprechendes Verfahren.

### Stand der Technik

In der Biochemie werden oftmals enzymatische Reaktionen durchgeführt. Enzymatische Reaktionen können beispielsweise im Zuge eines Aufreinigungsprotokolls für ein bestimmtes Protein oder beispielsweise bei der Synthese und/oder Vervielfältigung von Nukleinsäuren eingesetzt werden. In der Molekularbiologie werden sogenannte Ligasen eingesetzt, um die Synthese von Nukleinsäuren (DNA oder RNA) zu katalysieren. Durch die Verwendung von sogenannten Synthasen kann die Herstellung bestimmter anderer Stoffe katalysiert werden. Weit verbreitet ist die Verwendung des Enzyms Lysozym zum Aufschluss von bakteriellen Zellen. Proteinasen, z.B. Proteinase K, werden verwendet, um Proteine zu verdauen, d.h. abzubauen. Die Proteinase K wird beispielsweise für den Abbau von Proteinen in Zelllysaten eingesetzt, um Nukleinsäuren freizusetzen und die Nukleinsäuren verfügbar zu machen.

Für die Reaktionsführung bei der Verwendung von derartigen Enzymen ist es oftmals erforderlich, den lysierenden oder synthetisierenden Prozess in definierter Weise zu beenden. Dies spielt insbesondere bei automatisierten Prozessen eine große Rolle. Es sind daher bereits Ansätze bekannt, die mit einem gekoppelten enzymatischen System arbeiten, bei dem das erstes Enzym beispielsweise eine lysierende oder synthetisierende Aktivität aufweist. Zusätzlich ist ein zweites Enzym vorgesehen, das die enzymatische Aktivität des ersten Enzyms beenden kann, indem es das erste Enzym verdaut oder anderweitig inhibiert. Beispielsweise kann als erstes Enzym ein Lyseenzym vorgesehen sein, um einen Zellaufschluss zu bewerkstelligen. Anschließend erfolgt ein Verdau mit Proteinase K, wobei das Lyseenzym abgebaut und damit inaktiviert wird. Für die Durchführung dieser enzymatischen Reaktionen werden die Enzyme in der Regel nacheinander zugesetzt, wobei das zweite Enzym erst zugegeben wird, wenn die durch das erste Enzym katalysierte enzymatische Reaktion im ausreichenden Maße abgelaufen ist. Bei einer gemeinsamen Inkubation mit beiden Enzymen würde beispielsweise die Proteinase K das Lyseenzym vorzeitig zerstören. Es sind bereits auch Ansätze bekannt, bei denen sowohl das erste Enzym als auch das zweite Enzym von Anfang an gemeinsam im Ansatz vorhanden sind. Um jedoch eine ausreichend enzymatische Aktivität des ersten Enzyms trotz der inhibierenden Wirkung des zweiten Enzyms sicherzustellen, muss in diesen Fällen das erste Enzym in großem Überschuss vorhanden sein.

### Offenbarung der Erfindung

### Vorteile der Erfindung

Das erfindungsgemäße Enzymsystem geht von einem System aus, das wenigstens ein erstes Enzym und wenigstens ein zweites Enzym umfasst, wobei das zweite Enzym zur Inaktivierung des ersten Enzyms vorgesehen ist bzw. als unerwünschten Nebeneffekt neben seiner eigentlichen Funktion im Reaktionsansatz das erste Enzym inaktiviert oder verdaut. Beispielsweise soll mit dem Enzymsystem gleichzeitig eine Lyse von Zellen und ein Verdau von Proteinen durchgeführt werden. Beide Enzyme oder gegebenenfalls mehrere erste Enzyme und/oder mehrere zweite Enzyme sind in einem Reaktionsansatz vertreten. Erfindungsgemäß ist es vorgesehen, dass die Aktivitäten der Enzyme und insbesondere die Aktivität des oder der zweiten Enzyms/e durch eine Temperierung gesteuert werden. Im Zuge der Reaktionsführung wird durch eine Temperaturänderung der Arbeitspunkt während der Reaktion so verschoben, dass das zweite Enzym erst zeitverzögert aktiviert wird. Die Inaktivierung des ersten Enzyms, beispielsweise durch einen Verdau durch das zweite Enzym, erfolgt dann erst zeitverzögert. Unter "aktiviert" ist in diesem Zusammenhang zu verstehen, dass das zweite Enzym auch bereits vor der Temperaturänderung eine gewisse Aktivität aufweisen kann. Doch erst durch die Temperaturänderung wird die enzymatische Aktivität des zweiten Enzyms dominant. Vor dieser Aktivierung des zweiten Enzyms kann das erste Enzym seine Funktion, also beispielsweise eine lysierende, eine DNA oder RNA abbauende oder eine synthetisierende Funktion, erfüllen. Die Steuerung der enzymatischen Aktivitäten durch die Temperaturführung erlaubt es, dass in einem Reaktionsansatz sowohl das erste als auch das zweite Enzym enthalten sein können. Durch die Temperierung wird lediglich die Aktivität der beiden Enzyme beeinflusst. Bei dem erfindungsgemäßen Enzymsystem wird insgesamt der Reaktionsverlauf so gesteuert, dass eine zeitverzögerte Inaktivierung des ersten Enzyms (oder der ersten Enzyme) durch das zweite Enzym in temperaturgesteuerter Weise erfolgt. Es handelt sich also dabei um ein System, bei dem mit einem Reaktionsansatz gearbeitet werden kann, ohne dass eine Zugabe von weiteren Enzymen oder Reagenzien erforderlich ist, sodass die Handhabung dieses Systems einfach und zeitsparend ist. Im Vergleich mit Ansätzen aus dem Stand der Technik ist es dabei möglich, die Konzentration des ersten Enzyms an die tatsächlich erforderliche enzymatische Aktivität dieses Enzyms anzupassen. Es ist nicht erforderlich, das erste Enzym im Überschuss einzusetzen, wie es bei einer gemeinsamen Inkubation mit Ansätzen aus dem Stand der Technik erforderlich ist. Durch den erfindungsgemäßen Ansatz kann damit gegebenenfalls kostenintensives Enzym eingespart werden. Weiterhin ist es bei dem erfindungsgemäßen Enzymsystem nicht erforderlich, das zweite, inaktivierende Enzym nach einer gewissen Zeitdauer dem Reaktionsansatz erst hinzuzufügen. Alle für die Reaktionen erforderlichen Reagenzien sind in dem Ansatz bereits vorhanden. Außer der entsprechenden Temperierung ist kein weiterer Aufwand erforderlich. Somit werden insgesamt durch das erfindungsgemäße Enzymsystem Zeit und Arbeitsaufwand eingespart. Die Regulierung der Temperatur ist in sehr einfacher Weise, beispielsweise mit einem Thermoblock, möglich. Eine solche Temperierung kann beispielsweise programmiert werden, sodass kein weiterer Arbeitsaufwand bei der Durchführung der enzymatischen Reaktionen anfällt. Das erfindungsgemäße Enzymsystem ist damit in besonderer Weise einer Automatisierung zugänglich, beispielsweise kann es in sehr einfacher Weise mit Einsatz eines Pipettierroboters oder beispielsweise in einem mikrofluidischen System durchgeführt werden.

In einer bevorzugten Ausgestaltung des erfindungsgemäßen Enzymsystems handelt es sich bei dem ersten Enzym um ein lysierendes Enzym, beispielsweise Lysozym, oder um ein synthetisierendes Enzym, beispielsweise eine Ligase. Bei dem ersten Enzym kann es sich beispielsweise auch um eine DNAse oder um eine RNAse handeln. Bei dem zweiten Enzym handelt es sich um ein verdauendes Enzym, insbesondere um eine Proteinase, die allgemein Proteine und damit auch das erste Enzym verdaut bzw. abbaut. Ein Beispiel für eine geeignete Proteinase ist beispielsweise die Proteinase K.

In einer bevorzugten Ausgestaltung des erfindungsgemäßen Enzymsystems wird die Aktivität des zweiten Enzyms dadurch gesteuert, dass das zweite Enzym bei einer ersten Inkubationstemperatur eine niedrigere Aktivität als bei einer zweiten Inkubationstemperatur, die höher als die erste Inkubationstemperatur ist, aufweist. Die Enzyme werden dabei vorzugsweise so gewählt, dass das Temperaturoptimum des zweiten Enzyms bei einer höheren Temperatur als das Temperaturoptimum des ersten Enzyms liegt. Mit dem Begriff "Optimum" wird hierbei ein Bereich beschrieben, in dem das Enzym seine maximale bzw. mehr als einen bestimmten Prozentsatz seiner maximalen Aktivität entfaltet. Dies schließt allerdings nicht aus, dass auch außerhalb des Optimums, also vor Erreichen oder bei Überschreiten einer bestimmten Temperatur bzw. eines Temperaturbereichs, ein gewisses Maß an enzymatischer Aktivität vorhanden ist. Es handelt sich bei dieser Ausgestaltung des erfindungsgemäßen Enzymsystems und bei einer solchen Reaktionsführung also nicht um eine sequenzielle Aktivierung der Enzyme im strengeren Sinne, da das zweite Enzym auch schon zu Beginn der Inkubation, also bei der ersten Inkubationstemperatur, ein gewisses Maß an Aktivität aufweist. Jedoch ist diese Aktivität im Vergleich mit der Aktivität des ersten Enzyms so weit herabgesetzt, dass der Verdau des ersten Enzyms so weit verlangsamt ist, dass das erste Enzym seine Funktion im Rahmen der gewünschten Reaktion erfüllen kann. Beispielsweise kann eine vom ersten Enzym bewirkte Lyse, Synthese oder ein Verdau noch quantitativ ablaufen, bevor das erste Enzym vom zweiten Enzym in der Phase mit der zweiten Inkubationstemperatur inaktiviert oder verdaut wird. Im Vergleich mit herkömmlichen Ansätzen, bei denen bereits beide Enzyme im Ansatz vorhanden sind, bei denen aber das erste Enzym in großem Überschuss vorhanden sein muss, kann bei dieser Ausgestaltung des erfindungsgemäßen Enzymsystems die Konzentration des ersten Enzyms deutlich geringer gewählt werden. Voraussetzung für diese Ausführungsform ist, dass sich die Aktivitäten des ersten Enzyms und des zweiten Enzyms bei der zu Beginn der Inkubation eingestellten Temperatur deutlich voneinander unterscheiden. Anders ausgedrückt muss die Aktivität des zweiten Enzyms bei der zu Beginn der Inkubation (erste Inkubationsphase) eingestellten Temperatur deutlich geringer sein als bei der gegen Ende der Inkubation (zweite Inkubationsphase) eingestellten Temperatur, während das erste Enzym bereits bei der zu Beginn der Inkubation eingestellten Temperatur eine ausreichende Aktivität aufweisen muss. Beispielsweise kann als erstes Enzym ein Enzym gewählt werden, das bereits bei Raumtemperatur eine hohe Aktivität zeigt. Das Temperaturoptimum des zweiten Enzyms sollte dann deutlich höher liegen, beispielsweise in einem Bereich zwischen 35°C und 60°C, so dass die Aktivität des zweiten Enzyms bei Raumtemperatur relativ gering ist. Beispielsweise erreicht das erste Enzym bereits bei Raumtemperatur eine Aktivität von 50%, während das zweite Enzym eine Aktivität von 50% erst bei einer Temperatur zwischen 35 und 60°C erreicht. Durch Anfahren dieser verschiedenen Temperaturniveaus kann zunächst das erste Enzym und dann das zweite Enzym seine Aktivität entfalten.

In einer weiteren Ausführungsform des erfindungsgemäßen Enzymsystems weisen das wenigstens erste Enzym und das wenigstens zweite Enzym jeweils ein unterschiedliches Optimum im Hinblick auf das chemische Milieu auf. Hierfür können beispielsweise Enzyme eingesetzt werden, die unterschiedliche Bedürfnisse im Hinblick auf bestimmte Salze oder Ionen oder allgemein im Hinblick auf Cofaktoren aufweisen. Der Cofaktor kann beispielsweise ein bestimmtes Metallion sein, das in Form eines Salzes bereitgestellt wird. In dieser Ausführungsform wird die Aktivität des zweiten Enzyms durch eine Änderung des chemischen Milieus im Reaktionsansatz gesteuert. Vorzugsweise wird als zweites Enzym ein Enzym eingesetzt, dessen Aktivität von einem Cofaktor abhängt. Der Cofaktor wird erst dann zur Verfügung gestellt, wenn das zweite Enzym seine Aktivität entfalten soll. Dies erfolgt durch eine temperaturabhängige Freisetzung des Cofaktors. Beispielsweise können geeignete Cofaktoren im Ansatz zunächst immobilisiert sein, beispielsweise durch Einbettung in ein Wachs. Bei einer Temperatur unterhalb der Schmelztemperatur des Wachses liegen die Cofaktoren in immobilisierter Form vor und sind im Ansatz nicht verfügbar. Erst wenn eine Temperatur oberhalb der Schmelztemperatur des Wachses eingestellt wird, schmilzt das Wachs und der Cofaktor oder die Cofaktoren werden freigesetzt und stehen für das zweite Enzym zur Verfügung. Ein Beispiel für einen geeigneten Cofaktor ist beispielsweise Ca²⁺, das für die Aktivität der Proteinase K erforderlich ist. Indem das Ca²⁺ erst zu Beginn der zweiten Inkubationsphase freigesetzt wird, kann die verdauende Aktivität der Proteinase K so lange hinausgezögert werden.

In einer weiteren Ausgestaltung des erfindungsgemäßen Enzymsystems liegt das wenigstens zweite Enzym in immobilisierter und/oder blockierter Form vor. Diese Immobilisierung und/oder Blockierung ist temperaturabhängig, sodass durch die Temperaturführung die Immobilisierung oder Blockierung aufgehoben werden kann und damit das zweite Enzym seine Aktivität entfalten kann. Beispielsweise kann das zweite Enzym in ein schmelzbares Wachs eingebettet sein. Das zweite Enzym kann erst aktiv werden, wenn das Wachs durch Einstellung einer höheren Temperatur oberhalb des Schmelzpunktes des Wachses schmilzt. Eine temperaturabhängige Blockierung des zweiten Enzyms kann beispielsweise durch die Interaktion mit einem blockierenden Antikörper erreicht werden. Durch Erhöhung der Temperatur kommt es zu einem Lösen der Bindung oder zu einer Denaturierung des Antikörpers, wodurch wiederum das zweite Enzym seine enzymatische Aktivität entfalten kann.

Die Erfindung umfasst weiterhin einen Kit zur Durchführung von enzymatischen Reaktionen, wobei der Kit das beschriebene Enzymsystem sowie vorzugsweise weitere Reagenzien für die Bereitstellung eines Reaktionsansatzes enthält. Bei diesen weiteren Reagenzien kann es sich beispielsweise um Cofaktoren oder blockierende Substanzen (z.B. Antikörper) handeln, die im Hinblick auf die Aktivierung des zweiten Enzyms in der beschriebenen Weise erforderlich sind. Weiterhin können auch Puffer oder Salze oder andere Reagenzien im Kit enthalten sein, die allgemein für die Durchführung der enzymatischen Reaktionen erforderlich sind.

In einer besonders bevorzugten Ausführungsform des erfindungsgemäßen Kits liegt wenigstens eines der Enzyme des Enzymsystems, insbesondere das zweite Enzym, und/oder wenigstens eines der Reagenzien, insbesondere ein oder mehrere Cofaktoren für das zweite Enzym, in immobilisierter Form vor. Diese Immobilisierung ist temperaturabhängig, sodass durch eine entsprechende Temperaturführung das jeweilige Enzym und/oder der jeweilige Cofaktor freigesetzt wird, wodurch die Aktivität des zweiten Enzyms sich entfalten kann. Für die Immobilisierung eignet sich in besonderer Weise eine Einbettung in ein schmelzbares Material, insbesondere in Wachs. Besonders bevorzugt ist Paraffinwachs, dessen Schmelzpunkt in einem weiten Bereich eingestellt werden kann. Somit kann durch die Wahl eines bestimmten Paraffinwaches die Temperatur vorgegeben werden, bei der das zweite Enzym seine Aktivität entfalten soll. Das schmelzbare Material zusammen mit dem eingeschlossenen Enzym oder dem eingeschlossenen Reagenz kann beispielsweise in Form von Kügelchen bereitgestellt werden. In einer anderen Ausgestaltung kann das schmelzbare Material mit dem eingeschlossenen Enzym oder dem eingeschlossenen Reagenz beispielsweise als Schicht, insbesondere als dünne Schicht, in einem Reaktionsgefäß des Kits vorliegen.

In einer weiteren Ausgestaltung des erfindungsgemäßen Kits umfasst das Kit wenigstens eine Substanz, die das zweite Enzym in temperaturabhängiger Weise blockiert. Bei dieser Substanz kann es sich beispielsweise um einen blockierenden Antikörper handeln, der bei einer bestimmten Temperatur, insbesondere bei einer erhöhten Temperatur, sich von dem Enzym löst oder der bei einer bestimmten Temperatur denaturiert, sodass das Enzym seine Aktivität entfalten kann.

Die Erfindung umfasst weiterhin ein Verfahren zur Durchführung von enzymatischen Reaktionen, wobei in einem Ansatz wenigstens ein erstes Enzym und wenigstens ein zweites Enzym eingesetzt werden und wobei das zweite Enzym zur Inaktivierung des ersten Enzyms vorgesehen ist. Erfindungsgemäß werden die Aktivitäten der Enzyme und insbesondere die Aktivität des zweiten Enzyms durch eine Temperierung gesteuert, wobei vorzugsweise durch eine Temperaturerhöhung eine dominierende Aktivität des zweiten Enzyms ausgelöst wird. Für dieses Verfahren kann insbesondere das beschriebene Enzymsystem oder der beschriebene Kit eingesetzt werden. Dieses Verfahren eignet sich sehr für eine Automatisierung. Es kann beispielsweise in einem mikrofluidischen System, insbesondere in einem planaren mikrofluidischen System in sehr vorteilhafter Weise durchgeführt werden, wobei bei einem derartigen System eine Variation der Temperatur in einfacher Weise realisiert werden kann. Das planare System kann beispielsweise mit seiner Grundfläche in Kontakt mit einem Heizer stehen, wodurch eine Wärmeübertragung besonders effizient erfolgen kann. Das erfindungsgemäße Verfahren und das Enzymsystem sowie der Kit können mit Vorteil für verschiedene Routinen in der molekularen Diagnostik eingesetzt werden. Durch die einfache Steuerung der Reaktionsabläufe über eine Einstellung der Temperatur ist das Verfahren in sehr kostengünstiger Weise und mit wenig Aufwand realisierbar. Zudem ist es durch die Wahl entsprechender Enzyme sehr breit einsetzbar.

Die Erfindung umfasst schließlich ein mikrofluidisches System, insbesondere ein planares mikrofluidisches System, das das beschriebene erfindungsgemäße Enzymsystem enthält und das in besonders vorteilhafter Weise für eine automatisierte Durchführung entsprechender enzymatischer Reaktionen geeignet ist.

Weitere Merkmale und Vorteile der Erfindung ergeben sich aus der nachfolgenden Beschreibung von Ausführungsbeispielen in Verbindung mit den Zeichnungen. Hierbei können die einzelnen Merkmale jeweils für sich oder in Kombination miteinander verwirklicht sein.

### Kurze Beschreibung der Zeichnungen

In den Zeichnungen zeigen:
- Fig. 1: schematische Darstellung der Reaktionsabläufe bei der Verwendung einer bevorzugten Ausgestaltung des erfindungsgemäßen Enzymsystems und
- Fig. 2: schematische Darstellung der Reaktionsabläufe bei der Verwendung einer weiteren bevorzugten Ausgestaltung des erfindungsgemäßen Enzymsystems.

### Beschreibung von Ausführungsbeispielen

In einer ersten Ausführungsform des erfindungsgemäßen Verfahrens umfasst das Enzymsystem wenigstens ein erstes und wenigstens ein zweites Enzym, die sich in ihrem Temperaturoptimum unterscheiden. Insbesondere entfaltet das erste Enzym seine maximale Aktivität oder zumindest eine relativ zur Aktivität des zweiten Enzyms hohe Aktivität bereits bei einer verhältnismäßig niedrigeren Temperatur, beispielsweise bei Raumtemperatur. Das zweite Enzym entfaltet seine maximale Aktivität bei einer deutlich höheren Temperatur, wobei die optimale Temperatur beispielsweise in einem Bereich zwischen 35°C und 60°C liegen kann. Der Reaktionsansatz enthält von Anfang an beide Enzyme. Das erste Enzym ist beispielsweise eine DNAse oder eine RNAse oder ein Lyseenzym, z.B. Lysozym. Das zweite Enzym ist z.B. eine Proteinase, die das erste Enzym verdaut und es damit inaktiviert. Während des Reaktionsverlaufs wird die Temperatur so verändert, dass zu Beginn der Inkubation (erste Inkubationsphase) die Aktivität des ersten Enzyms im Vergleich zur Aktivität des zweiten Enzyms relativ hoch ist. Später in einer zweiten Inkubationsphase liegen die Reaktionsbedingungen näher beim Optimum des zweiten Enzyms. Die erste Inkubationsphase wird beispielsweise bei Raumtemperatur, je nach Enzym, durchgeführt, wobei dies der Temperaturbereich ist, bei dem die enzymatische Aktivität des ersten Enzyms dominiert. Für die zweite Inkubationsphase wird die Temperatur erhöht, wobei beispielsweise eine Temperatur zwischen 35°C und 60°C angefahren wird, also der Temperatur, bei der die enzymatische Aktivität des zweiten Enzyms dominiert. Die Dauer der Inkubationsphasen kann je nach eingesetzten Enzymen beispielsweise zwischen 1 und 30 Minuten, z. B. 10 Minuten, betragen. Die Inkubationsphasen können durch kontinuierliche Veränderung der Temperatur fließend ineinander übergehen (Temperaturrampe) oder die Temperaturstufen können direkt angefahren werden. Zur Einstellung der Temperatur kann das entsprechende Reaktionsgefäß beispielsweise in einen Thermoblock eingesetzt werden oder in anderer Weise temperiert werden.

Die enzymatischen Reaktionen können mit besonderem Vorteil in einem planaren mikrofluidischen System durchgeführt werden. Unter einem solchen System ist insbesondere ein polymerbasiertes System zu verstehen, das aus mehreren Schichten aufgebaut ist. Die laterale Ausdehnung eines solchen Systems ist deutlich größer als deren Dicke. Die Dicke der Schichten kann beispielsweise zwischen 50 µm und 10 mm betragen. Das System ist durch entsprechende Strukturierung der Schichten so ausgestaltet, dass Kanäle und Reaktionsräume in mikrofluidischem Maßstab gebildet werden, sodass die enzymatischen Reaktionen im mikrofluidischen Maßstab in automatisierter Weise durchgeführt werden können. Die Reaktionskammer kann beispielsweise als flache Kammer über einer Grundfläche des Systems ausgebildet sein. Diese Grundfläche kann mit einem Heizer in Kontakt gebracht werden, wodurch die Wärmeübertragung besonders effizient erfolgen kann und die erforderlichen Temperaturniveaus sehr schnell und effektiv erreicht werden können.

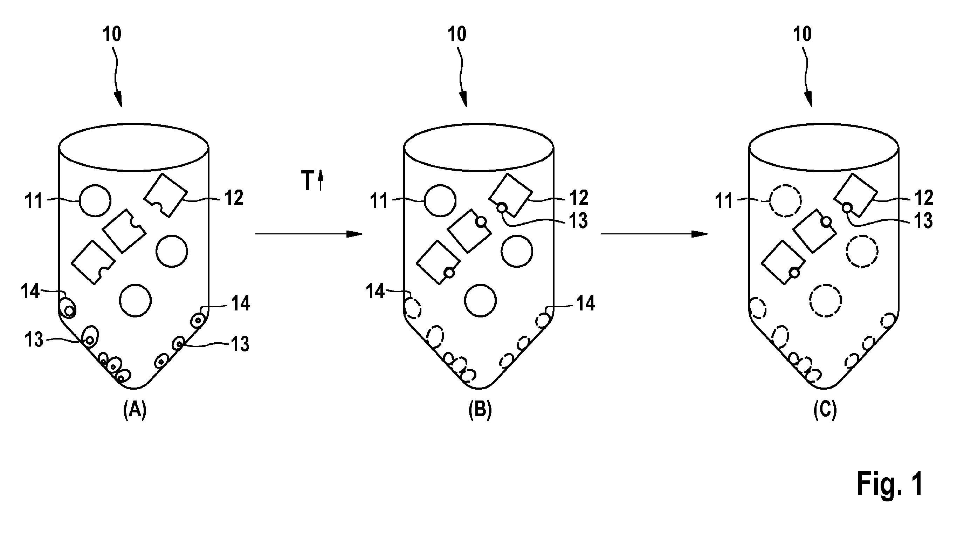

Eine weitere Ausgestaltung des erfindungsgemäßen Enzymsystems ist in **Fig. 1** illustriert. In dieser Variante der Erfindung werden ein erstes Enzym 11 und ein zweites 12 Enzym eingesetzt, deren Aktivität in unterschiedlicher Weise von einem Cofaktor 13, der beispielsweise in Form eines Salzes bereitgestellt wird, abhängt. Durch Veränderung des chemischen Milieus im Reaktionsansatz 10 im Hinblick auf diesen Cofaktor können die Aktivitäten der Enzyme gesteuert werden. Nur für die enzymatische Aktivität des zweiten Enzyms 12, also des verdauenden Enzyms, ist der Cofaktor 13 zwingend erforderlich. Der Cofaktor 13 wird erst im Verlauf der Inkubation durch Veränderung der Temperatur freigesetzt. In Teilabbildung (A) der Fig. 1 ist die Ausgangssituation beim Start der Reaktion gezeigt. Der Ansatz 10 wird beispielsweise bei Raumtemperatur inkubiert. Hierbei ist das erste Enzym 11, beispielsweise eine DNAse oder eine RNAse, enzymatisch aktiv. Das zweite Enzym 12 benötigt für eine enzymatische Aktivität den Cofaktor 13, der noch nicht zur Verfügung steht. Daher ist das Enzym 12 in der Phase (A) inaktiv bzw. nur relativ schwach aktiv. Beispielsweise handelt es sich bei dem zweiten Enzym 12 um eine Proteinase K, für deren Aktivität Ca²⁺ als Cofaktor erforderlich ist. Der Cofaktor 13, der in Form des Salzes CaCl₂ bereitgestellt wird, ist bereits im Reaktionsansatz 10 vorhanden. Der Cofaktor 13 befindet sich aber in immobilisierter Form 14 im Ansatz, wobei der Cofaktor 13 in ein Wachs eingebettet ist und in Form von Wachskügelchen 14 vorliegt. Der Durchmesser der Kügelchen 14 beträgt beispielsweise zwischen 100 µm und 5 mm. In einer alternativen Ausgestaltung kann der Cofaktor in einer dünnen Wachsschicht, beispielsweise mit einer Dicke zwischen 100 µm und 5 mm, eingebettet sein. Die Wachsschicht kann im Inneren des Reaktionsgefäßes, beispielsweise an den Wänden des Reaktionsgefäßes, angelagert sein. Das Wachs ist beispielsweise ein Paraffinwachs. Die Schmelzpunkttemperatur des Wachses liegt höher als die Temperatur in der ersten Inkubationsphase (A). Nach der ersten Inkubationsphase erfolgt eine Temperaturerhöhung bzw. ein Aufheizen auf eine zweite Temperatur, die oberhalb des Schmelzpunktes des Wachses liegt. Hierdurch wird das Schmelzen der Wachskügelchen 14 in der Phase (B) ausgelöst. Der Cofaktor 13 wird freigesetzt und kann mit dem Enzym 12 in Kontakt treten bzw. damit interagieren, sodass die Voraussetzung für die enzymatische Aktivität des zweiten Enzyms 12 gegeben ist. In der folgenden Phase (C), der zweiten Inkubationsphase, dominiert die enzymatische Aktivität des zweiten Enzyms 12, wodurch der Abbau bzw. der Verdau des ersten Enzyms 11 erfolgt.

Das Einbetten der Cofaktoren in das Wachs kann z.B. durch Verkneten des Cofaktors mit dem Wachs erfolgen. Hierfür wird das Wachs leicht erwärmt oder erweicht. In einer alternativen Ausgestaltung kann für die Einbettung der Cofaktoren das Wachs aufgeschmolzen und mit dem Cofaktor vermischt werden. Anschließend kann eine Ausformung von Kügelchen oder eine entsprechende Beschichtung des Reaktionsgefäßes mit dem immobilisierten Cofaktor erfolgen.

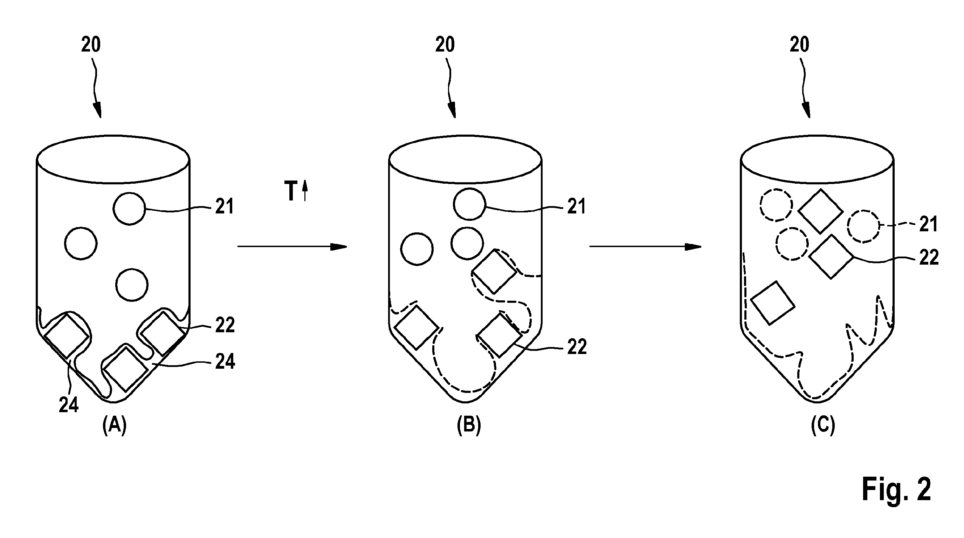

**Fig. 2** illustriert eine weitere Variante des erfindungsgemäßen Enzymsystems. Hierbei ist in dem Reaktionsansatz 20 das erste Enzym 21 und das zweite Enzym 22 enthalten, wobei das zweite Enzym 22 in der Phase (A) in einer Wachsschicht 24 eingebettet ist, sodass das zweite Enzym 22 in immobilisierter Form vorliegt. Das zweite Enzym 22 ist dabei inaktiv, sodass die erste Inkubationsphase mit der enzymatischen Aktivität des ersten Enzyms 21 ablaufen kann. Durch eine Temperaturerhöhung nach Beendigung der ersten Inkubationsphase, bei der nur das erste Enzym 21 aktiv ist, wird eine Temperatur oberhalb des Schmelzpunktes des Wachses eingestellt, sodass das zweite Enzym 22 freigesetzt wird (Phase (B)). Das zweite Enzym 22 kann nun seine enzymatische Aktivität entfalten. Das erste Enzym 21 wird verdaut und damit inaktiviert (Phase (C)). Ein besonderer Vorteil dieser Variante der Erfindung ist, dass während der ersten Inkubationsphase eine Aktivität des zweiten Enzyms mit besonders großer Zuverlässigkeit ausgeschlossen werden kann.

In einer weiteren Variante der Erfindung ist das zweite Enzym während der ersten Inkubationsphase an eine blockierende Substanz, insbesondere an einen blockierenden Antikörper gebunden. Dieser Antikörper kann insbesondere das aktive Zentrum des zweiten Enzyms besetzen, sodass die enzymatische Aktivität des zweiten Enzyms inhibiert ist. Durch eine Temperaturerhöhung nach Durchlaufen der ersten Inkubationsphase kommt es zu einem Lösen der Bindung oder zu einer Denaturierung des blockierenden Antikörpers, sodass das zweite Enzym aktiviert wird.

## Patentansprüche

1. Enzymsystem mit wenigstens einem ersten Enzym (11; 21) und wenigstens einem zweiten Enzym (12; 22), wobei die enzymatische Aktivität des zweiten Enzyms (12; 22) eine Inaktivierung des ersten Enzyms (11; 21) bewirkt, **dadurch gekennzeichnet, dass** die Aktivität des zweiten Enzyms (12; 22) durch eine Temperierung eines Reaktionsansatzes (10; 20), der das Enzymsystem enthält, steuerbar ist.

2. Enzymsystem nach Anspruch 1, **dadurch gekennzeichnet, dass** das erste Enzym (11; 21) ein lysierendes Enzym, ein DNA oder RNA abbauendes Enzym oder ein synthetisierendes Enzym ist und dass das zweite Enzym (12; 22) ein verdauendes Enzym, insbesondere eine Proteinase, ist.

3. Enzymsystem nach Anspruch 1 oder Anspruch 2, **dadurch gekennzeichnet, dass** das zweite Enzym (12; 22) bei einer ersten Inkubationstemperatur eine niedrigere Aktivität als bei einer zweiten Inkubationstemperatur, die höher als die erste Inkubationstemperatur ist, aufweist.

4. Enzymsystem nach einem der vorhergehenden Ansprüche, **dadurch gekennzeichnet, dass** die Aktivität des zweiten Enzyms (12; 22) durch eine Änderung des chemischen Milieus steuerbar ist.

5. Enzymsystem nach einem der vorhergehenden Ansprüche, **dadurch gekennzeichnet, dass** das zweite Enzym (22) in immobilisierter Form vorliegt, wobei die Immobilisierung temperaturabhängig ist.

6. Kit zur Durchführung von enzymatischen Reaktionen, **dadurch gekennzeichnet, dass** der Kit ein Enzymsystem gemäß einem der Ansprüche 1 bis 5 sowie vorzugsweise Reagenzien für die Bereitstellung eines Reaktionsansatzes (10; 20) enthält.

7. Kit nach Anspruch 6, **dadurch gekennzeichnet, dass** das zweite Enzym (22) des Enzymsystems und/oder wenigstens eines der Reagenzien (13), insbesondere ein Cofaktor für das zweite Enzym, in immobilisierter Form vorliegt, wobei die Immobilisierung temperaturabhängig ist, wobei vorzugsweise das zweite Enzym (22) und/oder das wenigstens eine Reagenz (13) in einem schmelzbaren Material, insbesondere in Wachs (14; 24), vorzugsweise Paraffinwachs, eingebettet ist.

8. Verfahren zur Durchführung von enzymatischen Reaktionen, wobei wenigstens ein erstes Enzym (11; 21) und wenigstens ein zweites Enzym (12; 22) eingesetzt werden und die enzymatische Aktivität des zweiten Enzyms (12; 22) eine Inaktivierung des ersten Enzyms (11; 21) bewirkt, **dadurch gekennzeichnet, dass** die Aktivitäten der Enzyme, insbesondere die Aktivität des zweiten Enzyms (12; 22), durch eine Temperierung, insbesondere durch eine Temperaturerhöhung, gesteuert werden.

9. Verfahren nach Anspruch 8, **dadurch gekennzeichnet, dass** ein Enzymsystem gemäß einem der Ansprüche 2 bis 5 und/oder ein Kit gemäß einem der Ansprüche 6 bis 8 eingesetzt wird.

10. Verfahren nach Anspruch 8 oder Anspruch 9, **dadurch gekennzeichnet, dass** das Verfahren in einem mikrofluidischen System, insbesondere in einem planaren mikrofluidischen System, durchgeführt wird.

11. Mikrofluidisches System, enthaltend ein Enzymsystem gemäß einem der Ansprüche 1 bis 5.
